# EUROPÄISCHE PATENTSCHRIFT

(11) **EP 0 377 905 B1**
(45) Veröffentlichungstag und Bekanntmachung des Hinweises auf die Patenterteilung: **16.02.1994**
(21) Anmeldenummer: 89124166.3
(22) Anmeldetag: 30.12.1989
(51) Int. Cl.: C07D 239/30

(54) **Verfahren zur Herstellung von 4,5,6-Trichlorpyrimidin**
Process for preparing 4,5,6-trichloropyrimidine
Procédé de fabrication de 4,5,6-trichloropyrimidine

(30) Priorität: 13.01.1989 DE 3900917
(43) Veröffentlichungstag der Anmeldung: 18.07.1990
(73) Patentinhaber: BAYER AG, 51368 Leverkusen (DE)
(72) Erfinder: Schündehütte, Karl-Heinz, Prof. Dr., D-5090 Leverkusen 3 (DE); Beck, Gunther, Dr., D-5090 Leverkusen 1 (DE); Findeisen, Kurt, Dr., D-5068 Odenthal 2 (DE); Henk, Hermann, Dr., D-5000 Köln 80 (DE)

(56) Entgegenhaltungen:
- EP-A- 0 249 257
- DE-A- 2 307 863
- FR-A- 1 545 313

## Beschreibung

Aus DE-A-23 07 863 ist ein Verfahren zur Herstellung von chlorierten Pyrimidinen bekannt, wobei N-(2-Cyanäthyl)formamide, die am N-Atom weiter durch einen unter den Reaktionsbedingungen abspaltbaren Rest substituiert sind, zunächst mit Säurechloriden bei 0-100°C und gleichzeitig, oder anschließend bei 0-250°C mit überschüssigem Chlor umgesetzt werden.

Gegenstand der vorliegenden Erfindung ist ein neues Verfahren zur Herstellung von 4,5,6-Trichlorpyrimidin, dadurch gekennzeichnet, daß man ein protoniertes Dialkylaminopropionitril der allgemeinen Formel I, 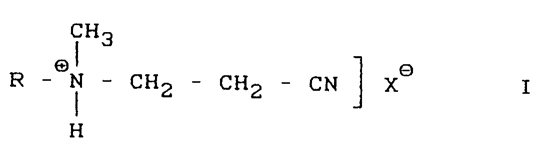
in der R ein Wasserstoff oder ein Alkylradikal mit 1 bis 4 C-Atomen bedeutet und X^{⊖} für ein Anion steht, in einem für die Reaktion inerten Lösungsmittel aus der Gruppe chlorierter aliphatischer und aromatischer Kohlenwasserstoffe, Phosphoroxychlorid, Isododecan und Chlorpyrimidinen in Anwesenheit von organischen Katalysatoren aus der Gruppe offenkettiger oder cyclischer Carbonamide, N-C₁-C₁₂-alkylpyrrolidone, N-C₁-C₁₂-alkylcaprolactame, Trialkylphosphite, Triarylphosphite und Triarylphosphinoxide, wobei Aryl vorzugsweise gegebenenfalls substituiertes Phenyl bedeutet, PCl₅ oder PCl₃ oder Gemische davon bei etwa 100 bis 150°C mit Chlor behandelt.

Bevorzugte Lösungsmittel sind solche, die unter Reaktionsbedingungen gegenüber Chlor stabil sind, z.B. Chloroform, Tetrachlorkohlenstoff, besonders aber Chlorbenzol und Dichlorbenzol sowie 4,5,6-Trichlorpyrimidin.

Besonders bevorzugt als Lösungsmittel ist Phosphoroxychlorid.

Geeignete Anionen sind insbesondere solche anorganischer starker Säuren wie Salzsäure oder Schwefelsäure. Daneben kommen auch Anionen organischer Säuren in Frage.

Geeignete organische Katalysatoren sind beispielsweise C₁-C₁₂-Dialkylformamide, insbesondere Dimethylformamid, Dibutylformamid, Methyldodecylformamid, N-Methylpyrrolidon-(2) und N-Methylcaprolactam.

Die Katalysatoren können auch im Gemisch untereinander eingesetzt werden, vorzugsweise in Mengen von 0,1 bis 30, vorzugsweise 10 bis 20 Gew.-%, bezogen auf I.

Die Reaktion wird vorzugsweise so durchgeführt, daß das Dialkylaminopropionitril in das vorgelegte Lösungsmittel gegeben wird und dann durch Zugabe von etwa 1,1 bis 1,3 Mol. Säure protoniert wird. Dabei erwärmt sich das Gemisch und i.a. fällt die Ammoniumverbindung als weißes, kristallines Salz aus. Dann werden 10 bis 20 Gew.-% des Katalysators zugegeben und die Suspension auf etwa 100 bis 130°C erwärmt. Anschließend wird bei dieser Temperatur zügig Chlor bis zur Beendigung der Reaktion eingeleitet. Die Gesamtdauer beträgt - je nach Katalysator - etwa 5 bis 20 Stunden. Das Reaktionsgemisch liegt dann als gelblich gefärbte Lösung vor. Für die Reaktion werden pro Mol freiem Ausgangsamin etwa 400 bis 600 g Chlor benötigt.

Anschließend kann das Gemisch destillativ getrennt werden. Das Reaktionsprodukt besteht aus praktisch reinem 4,5,6-Trichlorpyrimidin, das gegebenenfalls mit geringen Mengen Tetrachlorpyrimidin verunreinigt ist, das ebenfalls durch fraktionelle Destillation abgetrennt werden kann.

Im Vergleich zu vorbekannten Verfahren (z.B. US-Patent 3 509 032) besitzt die Synthese nach dieser Erfindung den Vorteil größerer Wirtschaftlichkeit bei gleichzeitig größerer Reinheit des entstandenen Produktes.

4,5,6-Trichlorpyrimidin ist ein interessantes Vorprodukt für die Reaktivkomponente 4,6-Difluor-5-chlor-pyrimidin (s. OS 1 644 203).

### Beispiel 1

98 g 3-Dimethylaminopropionitril werden in 600 ml Phosphoroxychlorid gelöst und durch Einleiten von 40 g Chlorwasserstoff (Dauer ca. 15 Minuten) das Hydrochlorid des 3-Dimethylaminopropionitril gebildet. In diese Suspension gibt man 15 g Phosphorpentachlorid, erwärmt auf Siedetemperatur (106°C) und leitet unter UV-Bestrahlung Chlor ein (ca. 45 bis 50 g pro Stunde). Nach 10 Stunden ist eine klare hellgelbe Lösung entstanden und es wurd kein Chlor mehr aufgenommen. Insgesamt wurden 465 g Chlor eingeleitet. Nach Beendigung der Chlorierung wird 30 Minuten mit Stickstoff gespült, dann das Phosphoroxychlorid abdestilliert und der Rückstand im Wasserstrahlvakuum destilliert bei 87 bis 125°C. Man erhält 178 g Destillat, welches zu 90,2 % aus 4,5,6-Trichlorpyrimidin und zu 9,1 % aus 2,4,5,6-Tetrachlorpyrimidin besteht.

Arbeitet man wie oben angegeben, verwendet jedoch als Katalysatoren die folgenden Verbindungen:
2. Dimethylformamid
3. Dibutylformamid
4. N-Methyl-pyrrolidon
5. N-Methyl-caprolactam
6. Trialkylphosphit
7. Triphenylphosphit
8. Triphenylphosphin
9. Triphenylphosphinoxid
10. Phosphorpentachlorid
so erhält man ähnliche Mengen an 4,5,6-Trichlorpyrimidin in vergleichbaren Reaktionszeiten.

### Beispiel 2

198 g (2 Mol) Dimethylaminopropionitril und 1105 g Chlorbenzol werden in einem 2 l Vierhalskolben mit Rührer, Gaseinleitungsrohr, Thermometer und absteigendem Kühler vorgelegt. Zuerst werden 95 g (2,6 Mol) Chlorwasserstoffgas eingeleitet. Die Temperatur steigt dabei von 20°C auf 90°C an, und das Hydrochlorid fällt weiß kristallin aus. Zu dieser Suspension gibt man dann 10 g Triphenylphosphin und 20 g Phosphorpentachlorid und leitet bei ca. 125°C innerhalb von 10 Stunden insgesamt 880 g trockenes Chlor gleichmäßig ein. Der entweichende Gasstrom enthält neben HCl und wenig Cl₂ Chloroform und Tetrachlorkohlenstoff. Nach Beendigung der Chlorierung wird 30 Minuten mit Stickstoff gespült und anschließend das Reaktionsgemisch fraktioniert destilliert. Nachdem das Chlorbenzol entfernt wurde, gehen 339 g 4,5,6-Trichlorpyrimidin bei einem Kp_{14mb}: 90 - 95°C über. Der Fp beträgt 51 - 52°C aus Petrolether. Ausbeute: 92,5 %.

Verfährt man wie in diesem Beispiel angegeben und verwendet als Katalysatoren folgende Verbindungen:
2. Dimethylformamid
3. Dibutylformamid
4. N-Methyl-pyrrolidon
5. N-Methyl-caprolactam
6. Trialkylphosphit
7. Triphenylphosphit
8. Triphenylphosphin
9. Triphenylphosphinoxid
10. Phosphorpentachlorid
so erhält man ähnliche Mengen an 4,5,6-Trichlorpyrimidin nach vergleichbaren Reaktionszeiten.

Analog kann die Synthese in o-Dichlorbenzol durchgeführt werden.

## Patentansprüche

1. Verfahren zur Herstellung von 4,5,6-Trichlorpyrimidin, dadurch gekennzeichnet, daß man ein protoniertes Dialkylaminopropionitril der allgemeinen Formel I, 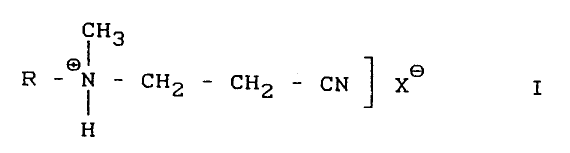 worin
R = H oder C₁-C₄-Alkyl
X^{⊖} = Anion
in Gegenwart organischer Katalysatoren aus der Gruppe offenkettiger und cyclischer Carbonamide, N-C₁-C₁₂-alkylpyrrolidone, N-C₁-C₁₂-alkylcaprolactame, Trialkylphosphite, Triarylphosphite und Triarylphosphinoxide, oder PCl₅ oder PCl₃ oder Gemische davon in einem für die Reaktion inerten Lösungsmittel aus der Gruppe chlorierter aliphatischer und aromatischer Kohlenwasserstoffe, Phosphoroxychlorid, Isododecan und Chlorpyrimidine bei einer Temperatur von etwa 100-150°C mit Chlor behandelt.

2. Verfahren nach Anspruch 1, dadurch gekennzeichnet, daß man als Katalysatoren Dimethylformamid, Dibutylformamid, N-Methylpyrrolidon, N-Methylcaprolactam, ein Tri-C₁-C₄-Alkylphosphit, Triphenylphosphin, Triphenylphosphinoxid oder PCl₅ bzw. Gemische dieser Verbindungen verwendet.

3. Verfahren nach Anspruch 1, dadurch gekennzeichnet, daß man als Lösungsmittel Phosphoroxychlorid oder Chlorpyrimidine verwendet.

4. Verfahren nach Anspruch 1, dadurch gekennzeichnet, daß man pro Mol Dialkylaminopropionitril 400 bis 600 g Chlor einsetzt.

## Claims

1. Process for preparing 4,5,6-trichloropyrimidine, characterized in that a protonated dialkylaminopropionitrile of the general formula I 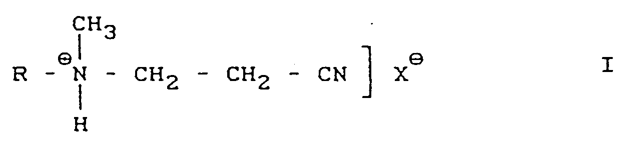 in which
R is H or C₁-C₄-alkyl
X⁻ is an anion
is treated with chlorine at a temperature of about 100-150°C in the presence of organic catalysts from the group consisting of open-chain and cyclic carboxamides, N-C₁-C₁₂-alkylpyrrolidones, N-C₁-C₁₂-alkylcaprolactams, trialkyl phosphites, triaryl phosphites and triarylphosphine oxides or PCl₅ or PCl₃, or mixtures thereof in a solvent which is inert for the reaction from the group consisting of chlorinated aliphatic and aromatic hydrocarbons, phosphorus oxychloride, isododecane and chloropyrimidines.

2. Process according to Claim 1, characterized in that the catalysts used are dimethylformamide, dibutylformamide, N-methylpyrrolidone, N-methylcaprolactam, a tri-C₁-C₄-alkyl phosphite, triphenylphosphine, triphenylphosphine oxide or PCl₅, or mixtures of these compounds.

3. Process according to Claim 1, characterized in that the solvent used comprises phosphorus oxychloride or chloropyrimidines.

4. Process according to Claim 1, characterized in that 400 to 600 g of chlorine are used per mole of dialkylaminopropionitrile.

## Revendications

1. Procédé de préparation de la 4,5,6-trichloropyrimidine, caractérisé en ce que l'on traite avec le chlore à une température d'environ 100 à 150°C un dialkylaminopropionitrile protoné de formule générale (I) : 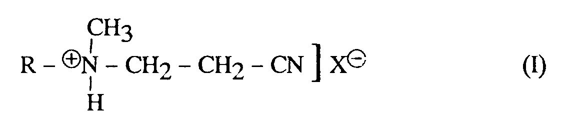 dans laquelle
R = H ou alkyle en C₁-C₄
X^{⊖} = anion
en présence de catalyseurs organiques du groupe des carbonamides à chaîne ouverte et cycliques, des N-alkyl-C₁-C₁₂-pyrrolidones, des N-alkyl-C₁-C₁₂-caprolactames, des phosphites de trialkyle, des phosphites de triaryle et des oxydes de triarylphosphine, ou de PCl₅ ou PCl₃ ou de leurs mélanges dans un solvant inerte pour la réaction, du groupe des hydrocarbures aliphatiques et aromatiques chlorés, de l'oxychlorure de phosphore, de l'isododécane et des chloropyrimidines.

2. Procédé selon la revendication 1, caractérisé en ce que l'on utilise comme catalyseurs le diméthylformamide, le dibutylformamide, la N-méthyl-pyrrolidone, le N-méthylcaprolactame, un phosphite de trialkyle en C₁-C₄, la triphénylphosphine, l'oxyde de triphénylphosphine ou PCl₅ ou des mélanges de ces composés.

3. Procédé selon la revendication 1, caractérisé en ce que l'on utilise comme solvant l'oxychlorure de phosphore ou des chloropyrimidines.

4. Procédé selon la revendication 1, caractérisé en ce que l'on utilise 400 à 600 g de chlore par mole de dialkylaminopropionitrile.
